# EUROPEAN PATENT APPLICATION

(11) **EP 4 070 726 A1**
(43) Date of publication of application: **12.10.2022**
(21) Application number: 21180984.3
(22) Date of filing: 22.06.2021
(51) Int. Cl.: A61B 5/11

(54) **POSTURE MEASUREMENT APPARATUS AND METHOD**

(30) Priority: 05.04.2021 IN 202111016012
(71) Applicant: NXP B.V., 5656 AG Eindhoven (NL)
(72) Inventor: Meijer, Rinze Ida Mechtildis Peter, 5656 AG Eindhoven (NL); Rajan Kesavelu Shekar, Pramod, 5656 AG Eindhoven (NL)
(74) Representative: Miles, John Richard

(57) **Abstract**

A posture measurement apparatus and method is described. The posture measurement system includes a wearable sensor leader node comprising a UWB transceiver coupled to at least two antennas and one or more wearable sensor follower nodes each comprising a UWB transceiver coupled to one antenna. A first UWB signal is transmitted from the wearable sensor leader node to the one or more wearable follower sensor nodes. A second UWB signal is received by the wearable sensor leader node from each follower sensor node in response to receiving the first UWB signal. A time-of-flight value of a signal transmitted between the wearable leader sensor node and the wearable follower sensor node is determined from the first UWB signal and the second UWB signal. An angle of arrival value is determined from the second UWB signal. The body posture can be determined from the time-of-flight and angle-of-arrival value.

## Description

### FIELD

This disclosure related to a method and apparatus for posture measurement using ultra-wide-band (UWB) transceivers.

### BACKGROUND

Over the last two decades, there has been a trend towards less physical activity has been due in part to the increased technological forms of work, and changing modes of transportation. This can result in poor body posture can have a detrimental effect on human health. In some countries, almost 80% of the population have experienced back pain at one point or another in their lives. The vast majority of back pain has no major trauma involved. It occurs from an accumulation of many minor injuries and years, even decades, of poor posture and bad postural habits. Being mindful of posture and making corrections as needed can help achieve proper posture with associated health benefits.

US9541994B2 describes a system comprising a sensor device which biomechanically detects in real-time a user's movement state and posture and then provides real-time feedback to the user based on the user's real-time posture. The sensor device detects the user's movement state and posture by capturing data from a tri-axial accelerometer in the sensor device. Streamed data from the accelerometer is normalized to correct for sensor errors as well as variations in sensor placement and orientation. Normalization is based on accelerometer data collected while the user is wearing the device and performing specific actions.

US20170156639A1 describes a system for mapping and monitoring posture that includes accelerometer sensor units that independently collect posture data corresponding to body locations or body positions on subjects and generate posture signals. The posture signals are wirelessly transmitted to one or more computers that are used to process the posture signals and generate posture feedback of the subjects in real-time.

### SUMMARY

Various aspects of the disclosure are defined in the accompanying claims. In a first aspect there is provided wearable leader sensor node for determining body posture, the wearable sensor leader node comprising: a processor; an orientation sensor coupled to the processor; a memory coupled to the processor; an ultra-wide-band, UWB, transceiver coupled to the processor; at least two antennas coupled to the UWB transceiver; wherein the processor is configured to: transmit a first UWB signal via the ultra-wide band transceiver to a wearable follower sensor node; receive a second UWB signal from the follower sensor node transmitted in response to receiving the first UWB signal; determine a time-of-flight value of a signal transmitted between the wearable leader sensor node and the wearable follower sensor node from the first UWB signal and the second UWB signal; determine an angle of arrival value of the second UWB signal; and determine a value representing a body posture from the time-of-flight value and the angle-of-arrival value.

In one or more embodiments, the processor may be configured to determine the body posture by comparison of the time-of-flight value and the angle of arrival value with a predetermined time-of-flight value and angle-of-arrival value stored in the memory.

In one or more embodiments, the processor is configured for each follower node to: transmit the first UWB signal via the ultra-wide band transceiver comprising a follower node ID; receive a second UWB signal from the follower sensor node having the follower node ID; determine a time-of-flight value of a signal transmitted between the wearable leader sensor node and the follower sensor node from the first UWB signal and the second UWB signal; determine an angle of arrival of the second UWB signal from the wearable follower sensor node; and determine a value representing a body posture from the time-of-flight values and the angle-of-arrival values determined between the sensor leader node and the follower nodes.

In one or more embodiments, the angle of arrival is determined with respect to a reference axis determined from the orientation sensor.

In one or more embodiments, the processor is further configured in a calibration step for each body posture to store the time-of-flight values and the angle-of arrival values in the memory.

In one or more embodiments, the wearable sensor leader node may be further configured to determine a user action from a comparison of the measured time-of-flight values and the angle-of arrival values with reference time-of-flight values and the angle-of arrival values.

In one or more embodiments, the wearable sensor leader node may further comprise: a radio frequency, RF, transceiver coupled to the processor wherein the processor is further configured to transmit the determined body posture value via the RF transceiver to a further device configured to indicate to a user a required change in body posture.

In one or more embodiments, the further device may be configured to indicate the required change in body posture from a difference between a measured angle-of-arrival value and a predetermined angle-of-arrival value.

Embodiments of the wearable leader sensor node may be included in a posture measurement apparatus further including a plurality of wearable follower sensor nodes, each wearable follower sensor node may comprise a processor; a memory coupled to the processor; an ultra-wide-band, UWB, transceiver coupled to the processor; an antenna coupled to the UWB transceiver; wherein the follower sensor node processor is configured to: receive a first UWB signal via the ultra-wide band transceiver from the wearable leader sensor node; transmit a second UWB signal to the wearable leader sensor node in response to receiving the first UWB signal.

In a second aspect, there is provided in a posture measurement system comprising a wearable sensor leader node, the wearable sensor leader node comprising a UWB transceiver coupled to at least two antennas and one or more wearable sensor follower nodes each comprising a UWB transceiver coupled to one antenna, a method of determining a body posture comprising: transmitting a first UWB signal from the wearable sensor leader node to the one or more wearable follower sensor nodes; receiving a second UWB signal by the wearable sensor leader node from each follower sensor node transmitted in response to receiving the first UWB signal; determining a time-of-flight value of a signal transmitted between the wearable leader sensor node and the wearable follower sensor node from the first UWB signal and the second UWB signal; determining an angle of arrival of the second UWB signal; and determining a value representing a body posture from the time-of-flight value and the angle-of-arrival value.

In one or more embodiments, the method may further comprise determining the body posture value by comparison of the time-of-flight value and the angle of arrival value with a predetermined time-of-flight value and angle-of-arrival value.

In one or more embodiments, the method may further comprise transmitting the first UWB signal from the wearable leader sensor node to a plurality of wearable follower sensor nodes;

receiving by the wearable leader sensor node a plurality of second UWB signals from each of the follower sensor nodes transmitted in response to receiving the first UWB signal by each of the respective follower sensor nodes ; determining a time-of-flight value of a signal transmitted between the wearable leader sensor node and each wearable follower sensor node from the first UWB signal; determining an angle of arrival of the second UWB signal from each of the wearable follower sensor nodes; and determining a value representing a body posture from the time-of-flight values and the angle-of-arrival values.

In one or more embodiments, the leader sensor node may comprise a orientation sensor and the method may further comprise: determining a reference axis form the orientation sensor; and determining an angle of arrival with respect to the reference axis.

In one or more embodiments, the method may further comprise in a calibration step determining reference values of the time-of-flight and the angle-of arrival.

In one or more embodiments, the method may further comprise determining a user action from a comparison of the measured time-of-flight values and the angle-of arrival values with the reference time-of-flight values and the reference angle-of arrival values.

In a third aspect, there is provided a non-transitory computer readable media comprising a computer program comprising computer executable instructions which, when executed by a computer, causes the computer to perform a method of determining body posture comprising:
transmitting a first UWB signal from a wearable sensor leader node to one or more wearable follower sensor nodes; receiving a second UWB signal by the wearable sensor leader node from each follower sensor node transmitted in response to receiving the first UWB signal; determining a time-of-flight value of a signal transmitted between the wearable leader sensor node and the wearable follower sensor node from the first UWB signal and the second UWB signal; determining an angle of arrival of the second UWB signal; and determining a value representing a body posture from the time-of-flight value and the angle-of-arrival value.

In one or more embodiments, the non-transitory computer readable media may further comprise computer executable instructions which, when executed by a computer, causes the computer to perform the steps of determining the body posture value by comparison of the time-of-flight value and the angle of arrival value with a predetermined time-of-flight value and angle-of-arrival value.

In one or more embodiments, the non-transitory computer readable media may further comprise computer executable instructions which, when executed by a computer, causes the computer to perform the steps of: transmitting the first UWB signal from the wearable leader sensor node to a plurality of wearable follower sensor nodes; receiving by the wearable leader sensor node a plurality of second UWB signals from each of the follower sensor nodes transmitted in response to receiving the first UWB signal by each of the respective follower sensor nodes ; determining a time-of-flight value of a signal transmitted between the wearable leader sensor node and each wearable follower sensor node from the first UWB signal and the respective second UWB signal; determining an angle of arrival of the second UWB signal from each of the wearable follower sensor nodes; and determining a value representing a body posture from the time-of-flight values and the angle-of-arrival values.

In one or more embodiments, the non-transitory computer readable media may further comprise computer executable instructions which, when executed by a computer, causes the computer to perform the steps of: determining a reference axis from an orientation sensor in the leader sensor node; and determining an angle of arrival with respect to the reference axis.

In one or more embodiments, the non-transitory computer readable media may further comprise computer executable instructions which, when executed by a computer, causes the computer to perform the steps of determining reference values of the time-of-flight and the angle-of arrival in a calibration step.

### BRIEF DESCRIPTION OF THE DRAWINGS

In the figures and description like reference numerals refer to like features. Embodiments are now described in detail, by way of example only, illustrated by the accompanying drawings in which:
Figure 1 shows a posture measurement system including UWB leader sensor device and a UWB follower sensor device according to an embodiment.
Figure 2A illustrates a posture measurement system showing the position of UWB leader and follower sensor devices according to an embodiment.
Figure 2B shows the representation of angles with respect to a reference axis between each follower node and leader node for a front view standing posture for the posture measurement system of figure 2A.
Figure 2C shows a lateral view of the representation of angles from each follower node to leader node for standing and sitting posture for the posture measurement system of figure 2A.
Figure 3 illustrates a posture measurement system showing the position of UWB leader and follower sensor devices according to an embodiment.
Figure 4 illustrates a method of posture measurement according to an embodiment.
Figure 5 shows a method of posture analysis for the posture measurement method of figure 4.
Figure 6 illustrates a method of posture measurement using a further device for posture analysis according to an embodiment.
Figure 7 shows an example transaction diagram for the posture measurement method of figure 6
Figures 8A and 8B show calibration method for embodiments of the posture measurement system.

### DETAILED DESCRIPTION OF EMBODIMENTS

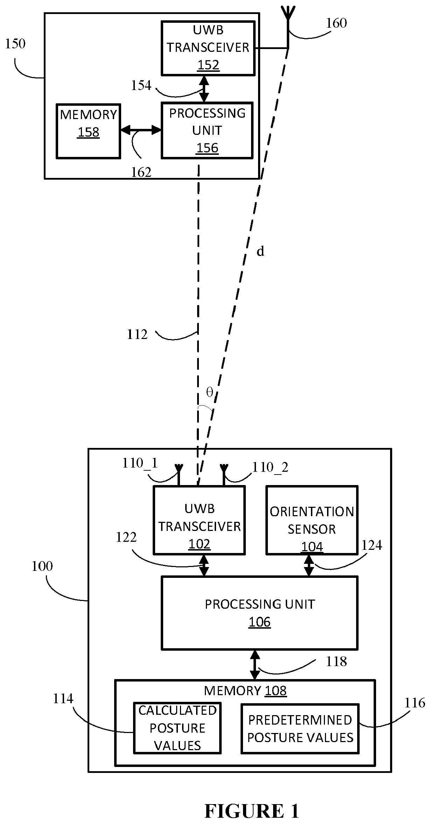

Figure 1 shows a UWB posture measurement system according to an embodiment. The UWB posture measurement system includes a body-worn leader sensor device 100 which may also be referred to as a wearable leader sensor node and a body-worn follower sensor device 150 may also be referred to as a wearable follower sensor node.

The leader sensor device 100 includes a UWB transceiver 102, an orientation sensor 104, a processing unit 106 and a memory 108. The UWB transceiver 120 may have a first and second antenna 110_1, 110 2. The UWB transceiver 102 may be coupled to the processing unit 106 via bidirectional connection 122. The orientation sensor 104 may be coupled to the processing unit 106 via the bidirectional connection 124. The memory 108 may include calculated posture values 114 which are posture values calculated during operation by the processing unit 106 and predetermined posture values 116. In some examples, the predetermined posture values 116 may be determined in a calibration step. In some examples, the predetermined posture values 116 may be stored in an a further device which may be an off-body device (not shown) . The orientation sensor 104 may be coupled to the processing unit 106 via a bidirectional connection 118. The orientation sensor 104 may be implemented using a gyro, an accelerometer or other suitable sensor.

The follower sensor device 150 includes a UWB transceiver 152, a follower processing unit 156 and a follower memory 158. The follower processing unit 156 may be implemented as software running on a microprocessor or hardware for example by a state machine. The UWB transceiver 152 may have an antenna 160. The UWB transceiver 152 may be coupled to the processing unit 156 via bidirectional connection 154. The follower memory 158 may be coupled to the processing unit 106 via a bidirectional connection 162.

In operation, the leader sensor device 100 may transmit a signal which may include a challenge packet generated by the processing unit 106 via the leader UWB transceiver 102. This UWB signal may then be received by the follower sensor device 150 via the follower UWB transceiver 152. The processing unit 156 of the follower sensor device 150 may generate a signal including a response packet transmitted via the follower UWB transceiver 152. In one example, the processing unit 156 may be implemented as a logic state machine which may read the follower node identifier (ID) from the memory 158 and transmit the follower node ID via the follower UWB transceiver 152. The UWB signal transmitted from the follower sensor device 150 may be received by the leader sensor device 100 via the UWB transceiver 102.

The leader sensor device 100 may then determine two values based on the received UWB signal. The first value may be the distance d between the leader sensor device 100 and the follower sensor device 150 corresponding to the distance between the antenna 160 of the follower sensor device 150 and the two antennas 110_1, 110_2 of the leader sensor device 100. This distance then may be determined using time-of-flight (ToF) ranging methods. Each UWB device may start ranging when the device is turned on by the user. The ToF is calculated by measuring the roundtrip time of challenge/response packets as explained for example in https://www.firaconsortium.org/discover/how-uwb-works. The second value is the angle of arrival θ which may be determined from the phase difference of the signal received at the first antenna 110_1 and the second antenna 110_2 of the UWB transceiver 102. This angle of arrival value θ may be determined with respect to a reference axis 112 which is determined by the processing unit 106 from the orientation sensor 104. The processing unit 106 may then determine from the distance and angle of arrival a value representing the posture of a user on which the leader sensor device 100 and the follower sensor device 150 is positioned. This calculated posture value 114 may be stored in memory 108. The calculated posture value 114 may then be compared with predetermined posture values 116 to determine whether the posture is correct or whether corrective action is required. In other examples the leader sensor node may have a RF transceiver (not shown) such as a Bluetooth or Wi-Fi transceiver coupled to the processing unit 106. In these examples the leader sensor node may transmit the distance and angle of arrival values to a further device (not shown) which may perform the posture analysis.

The inventors of the present disclosure have appreciated that by using UWB signals for ranging and angle of arrival, the relative location of each sensor node on the body may be determined with high accuracy. Furthermore a reference axis measurement may be determined by a single orientation sensor device located in only one other sensor nodes i.e. the leader sensor device node 100. The posture measurement system including the leader sensor device node 100 and follow sensor device 150 may be scaled by including further follow sensor devices which may be positioned at various points on a user's body to determine more accurately the posture or movement of a user of the posture measurement system.

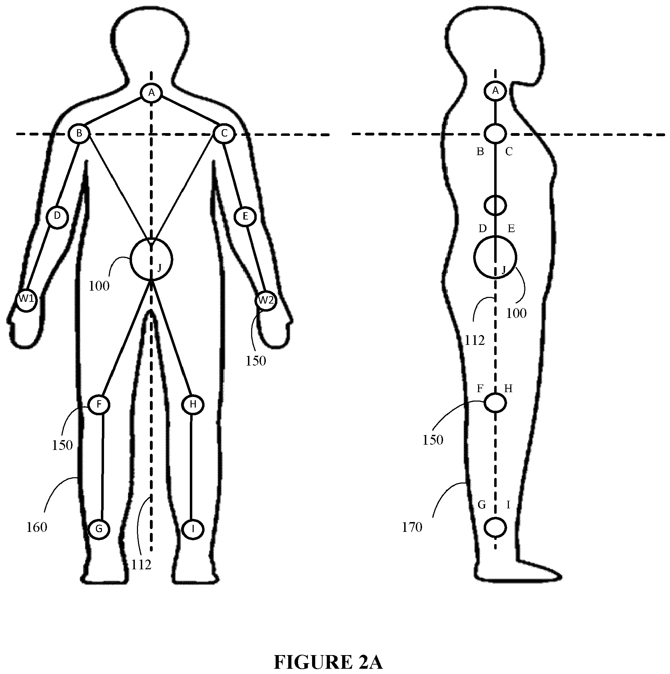

This scalability is illustrated further in figure 2A which shows a posture measurement system according to an embodiment positioned on a user. The front view of the user 160 shows follower sensor devices 150 positioned at nodes A-I, W1, W2. The leader sensor device 100 may be positioned at node J approximately in the middle of the lower back. In other examples, the leader sensor device 100 may be positioned at other locations on the body. Node A is located at the neck, nodes B and C are located at the shoulders, nodes D and E are located at the elbows, nodes W1 and W2 are located at the wrists, nodes F and H are located at the knees, and nodes G and I located at the ankles. The lateral view 170 of the user of the posture measurement system shows the equivalent location seen from the side.

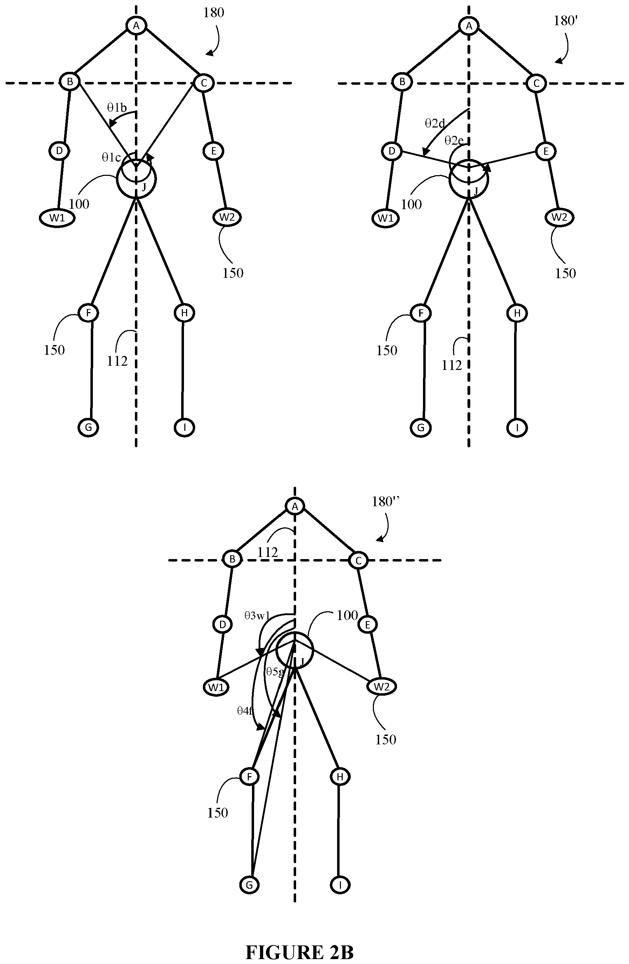

Ranging and position from each UWB node A,B,C,D,E,F,G,H,I,W1,W2 is calculated by the leader sensor device 100 at the base Node J. A reference axis 112 is determined by the processing unit 106 from the orientation sensor 104 at Node J. The angle calculation from each Node is computed at Node J with respect to the reference axis 112 using an angle of arrival calculation as previously described. Figure 2B shows the representation of angular details obtained from each node to the base Node J from the reference axis for standing posture. Referring to the front view 180, angle θ1 is the angle determined from the shoulder nodes. As illustrated, θ1b and θ1c are the angles determined from the respective shoulder nodes B, C to the reference axis 112.

Referring to the front view 180', angle θ2 is the angle computed at Node J from the nodes of elbow D and E with the reference axis 112. As illustrated θ2d is the angle between node D and node J and θ2e is the angle between node E and node J. Referring to the front view 180", angle θ3 is the angle computed at Node J from the nodes of Wrist W1 and W2 with respect to the reference axis 112. As illustrated θ3w1 is the angle between node W and node J. Angle θ4 is the Angle computed at Node J from the nodes of Thigh/knee F and H with the reference axis. As illustrated θ4f is the angle between node F and node J. Angle θ5 is the Angle computed at Node J from the nodes of ankle G and I with respect to the reference axis 112. As illustrated θ5g is the angle between node G and node J.

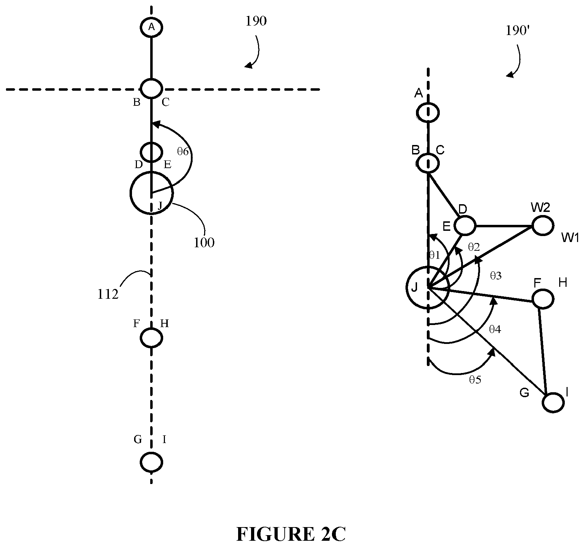

Figure 2C shows the lateral standing view 190. The angle θ6 is the angle computed at Node J from the node A at the neck with respect to the reference axis 112.

Figure 2C shows the equivalent angles θ1- θ5 as described for figure 2B shown as a lateral view 190' of a person in a seated position.

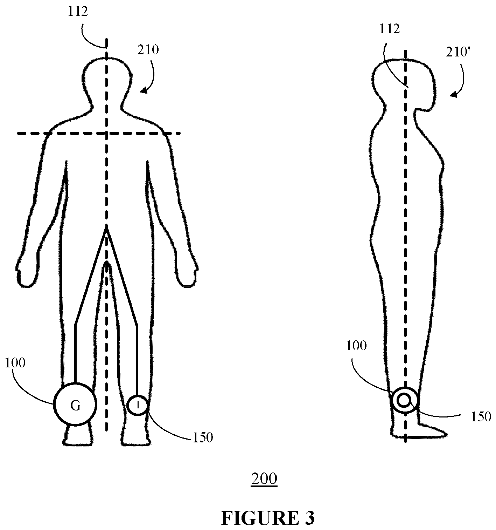

Figure 3 shows a posture measurement system 200 including a single leader device 100 and a single follower device 150 according to an embodiment. The leader device 100 and follower device 150 are located on the ankles of a user as shown in front view 210 and lateral view 210'. The angular details and computation is performed only for the two nodes and the feedback is provided to the user. The user activity which may be monitored may be for example cadence and/or cycling.

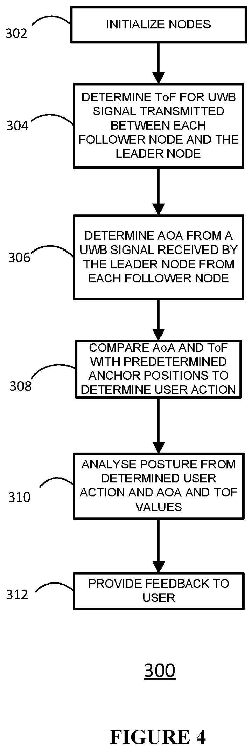

Figure 4 shows a method of posture measurement 300 according to an embodiment. The method 300 may be used for example by the posture measurement systems illustrated in figures 1, 2A, and 3. In step 302 the nodes may be initialised. A node may consist of either a follower sensor device, such as follower sensor device 150 or a leader sensor device, such as leader sensor device 100. As previously explained only one leader sensor device is required for posture measurement. Each of the follower sensor devices and leader sensor devices include a UWB transceiver. The leader sensor device also includes an orientation sensor. Initialization of all the nodes with respect to the orientation sensor may determine the reference axis and the position of each nodes on the body when the user turns on the device.

In step 304 a time-of-flight may be determined for a UWB signal transmitted between each follower node and the leader node. In step 306 an angle of arrival may be determined from a UWB signal received by the leader node from each follower node. In step 308 the angle of arrival and time-of-flight may be compared with predetermined anchor positions to determine a user action, such as to determine whether a person is standing or sitting. For example with reference to figures 2B and 2C, the determination of whether a user is standing or setting may be calculated from the angles θ3, θ4, θ5, θ6. In step 310, the posture may be analysed based on the determined user action and the angle of arrival and time-of-flight values. Following step 310, in step 312 relevant corrective feedback may be provided to the user for corrective posture to be maintained.

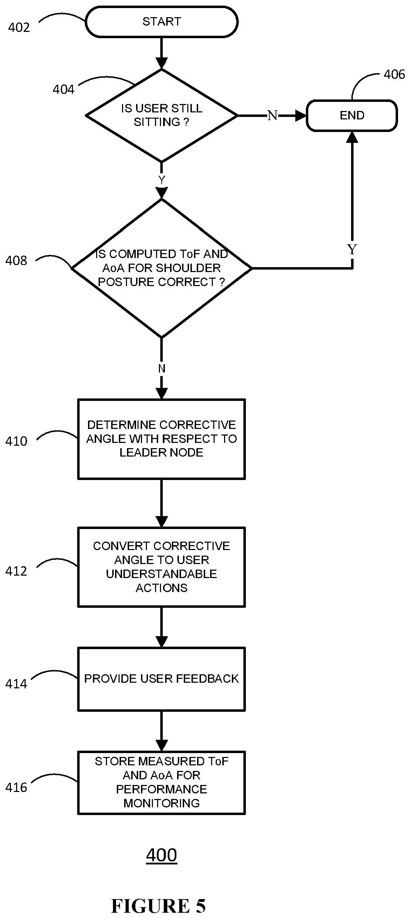

Figure 5 shows a method of posture analysis 400 which may be used for example to implement step 310 of the method 300. In step 402 the method starts. In step 404 a check is made to determine whether the user is still sitting. If the user is still sitting, the method ends in step 406. Returning to step 404 if the user is not sitting in the method moves to step 408 and a comparison is made between the computed time-of-flight and computed angle of arrival for shoulder posture and the correct angular posture to determine whether or not the posture is correct. If the posture is correct and the method ends in step 406. The posture is not correct then in step 410 the corrective angle is determined with respect to the leader node. In step 412 the corrective angle is converted to use understandable actions. In step 414 user feedback is then provided to correct the posture. In step 416 the measure time-of-flight and angle of arrival may be stored for performance monitoring. Method steps 408 to 412 may be repeated for each different measured angle, for example angles θ1-θ6 in figure 2B. Method 400 is shown for a sitting position but it will be appreciated that the methods may be used for different user actions such as standing, walking and running.

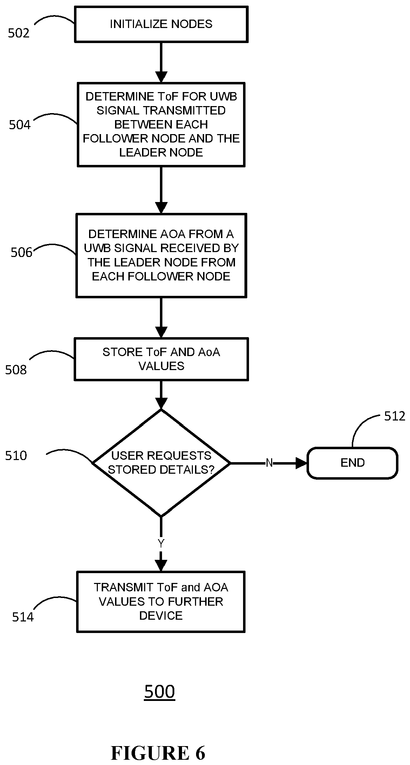

Figure 6 shows a method of posture measurement 500 according to an embodiment. In step 502 the nodes may be initialized. In step 504 a time-of-flight may be determined for a UWB signal transmitted between each follower node and the leader node. In step 506 an angle of arrival may be determined from a UWB signal received by the leader node from each follower node. In step 508 the time-of-flight and angle of arrival values may be stored for example in the memory 108 of the leader sensor device 100. In step 510 a check may be made on whether the user has requested the stored details. If the user has not requested stored details then the method ends at step 512. If the user has requested stored details, then in step 514 the time-of-flight and angle of arrival information may be transmitted to a further device which may be a device such as a smart watch, a mobile phone or some other device. In this example the leader node may include an additional transceiver such as a Bluetooth transceiver which may be used to communicate with a further device. The further device may then calculate the posture of the user, for example using steps 308 to 312 in method 300.

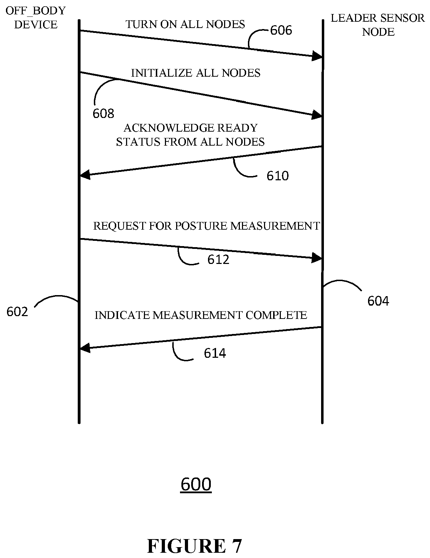

Figure 7 shows an example communication sequence 600 between a leader sensor device 604 to a further "off-body" device 602 such as a smart watch or mobile device. In step 606 the user turns on all the nodes which may be either using a switch or via an event detected by the off-body device. In step 608 a request may be sent to initialize all the bodes on the body. In step 610 the nodes may indicated their ready status. The ready status may be a light indicator or acknowledgement signal transmitted from all the nodes to the leader node. The ready status may also include the number of nodes active/present.

After the successful acknowledgement in step 610, in step 612 the user may request that the posture measurement starts. The user may determine the intervals or specific period of time to perform the analysis. The user may determine the profile for measurement during the activity. A use case scenario may be static, such as standing or sitting, or dynamic such as walking or running. For the static use case scenario, a one-time measurement may be sufficient to perform the posture analysis. For the dynamic use-case scenario, the user may determine the period of intervals at which the analysis is to be made. For example, while walking the user can set the posture analysis interval to 10ms. The status may be indicated to the user by the leader node in step 614 after the measurement and analysis is completed.

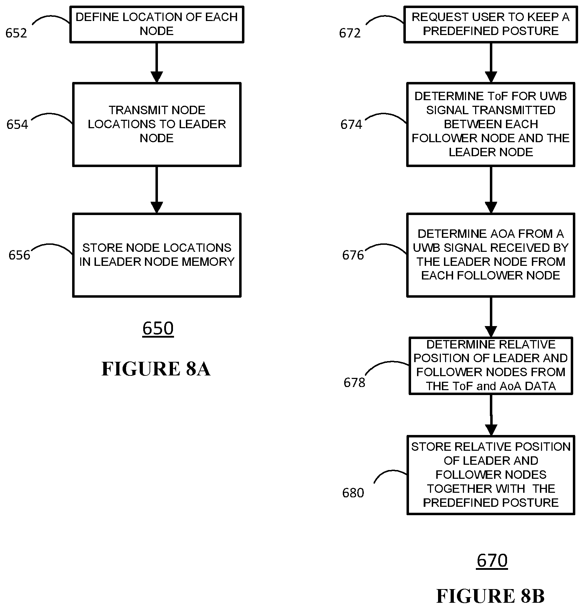

Figures 8A and Figure 8B show example calibration steps which may be used as a one-off calibration or more precede the initialization steps for example step 302 of method 300 and step 502 of method 500. The initialization may for example be a user-calibration or self-calibration.

Figure 8A shows a method of user controlled calibration 650. In step 652 a person may define the location of each sensor node using an application program which may for example be implemented on an off-body device. For example, the leader node is assigned to lower-back position, a first follower sensor node is assigned to left-shoulder position, a second follower sensor-node is assigned to right-shoulder position, etcetera. The identification of each sensor node could be done by visual means, or electronic means for example with a unique ID.

In step 654 the node assignment can be applied, and the information may be transmitted from the off-body device (with known sensor node positions) to the leader sensor node. In step 656, these locations are then stored in the memory of the leader sensor node, i.e. this may be stored in 116 in Figure 2. This completes the user calibration method 650.

Figure 8B shows a method of self-calibration 670. In step 672, a user (person) may be requested to keep a particular position during the self-calibration step. For example, for self-calibration the person may be requested to stand up straight or to perform a predefined activity for a predefined time for example by walking around for a number of seconds.

The leader sensor node is the initiator of the calibration sequence. In step 674, the leader sensor node requests each follower sensor node to send data to the leader sensor node, similarly to the regular operation. Based on the received data from a follower sensor node, the leader sensor node determines the time of flight of the related sensor node in step 674 and the angle of arrival in step 676. This process is completed once each follower sensor node has provided the data, and the leader sensor node has determined its position.

In some examples an off-body device may provide input on the number of sensor node to be included for the self-calibration step, for example, eight body worn sensor nodes. This may provide a reference to the leader sensor node on the number of sensor nodes to be included in the calibration step. In step 678 the relative position of the leader and follower nodes may be determined from the time of flight and angle of arrival values. In step 680, the relative position of the leader nodes together with the predefined posture may be stored in the leader node memory.

In some examples, the self-calibration step can also be initiated by the off body device. In this case, the command is provide by the off-body device to the leader sensor node, and the leader sensor node may then communicate with all follower sensor nodes.

Embodiments described in the present disclosure may allow improved accuracy of posture measurement. Further because the system only requires one leader device, the posture measurement system may be scaled by adding follower sensor devices to further improve the accuracy of the posture measurement. Some embodiments may be used to analyse the posture of patients by doctors or physiotherapists. Other embodiments may be used by individuals to measure their own postural information and take corrective actions for improvement. In other examples, the posture measurement system may be used for sports & fitness, to receive corrective feedback during activities. This corrective feedback may enable performance monitoring and tracking of individual's posture activity to implement other measurements than posture such as cadence, step measurement of the user, or fitness related monitoring such as checking whether exercises such as bench press are being performed correctly.

Embodiments may be included in products implemented as body-worn patches, body-worn activity monitoring devices, or smart clothing.

A posture measurement apparatus and method is described. The posture measurement system includes a wearable sensor leader node comprising a UWB transceiver coupled to at least two antennas and one or more wearable sensor follower nodes each comprising a UWB transceiver coupled to one antenna. A first UWB signal is transmitted from the wearable sensor leader node to the one or more wearable follower sensor nodes. A second UWB signal is received by the wearable sensor leader node from each follower sensor node in response to receiving the first UWB signal. A time-of-flight value of a signal transmitted between the wearable leader sensor node and the wearable follower sensor node is determined from the first UWB signal and the second UWB signal. An angle of arrival value is determined from the second UWB signal. The body posture can be determined from the time-of-flight and angle-of-arrival value.

In some example embodiments the set of instructions/method steps described above are implemented as functional and software instructions embodied as a set of executable instructions which are effected on a computer or machine which is programmed with and controlled by said executable instructions. Such instructions are loaded for execution on a processor (such as one or more CPUs). The term processor includes microprocessors, microcontrollers, processor modules or subsystems (including one or more microprocessors or microcontrollers), or other control or computing devices. A processor can refer to a single component or to plural components.

In other examples, the set of instructions/methods illustrated herein and data and instructions associated therewith are stored in respective storage devices, which are implemented as one or more non-transient machine or computer-readable or computer-usable storage media or mediums. Such computer-readable or computer usable storage medium or media is (are) considered to be part of an article (or article of manufacture). An article or article of manufacture can refer to any manufactured single component or multiple components. The non-transient machine or computer usable media or mediums as defined herein excludes signals, but such media or mediums may be capable of receiving and processing information from signals and/or other transient mediums.

Example embodiments of the material discussed in this specification can be implemented in whole or in part through network, computer, or data based devices and/or services. These may include cloud, internet, intranet, mobile, desktop, processor, look-up table, microcontroller, consumer equipment, infrastructure, or other enabling devices and services. As may be used herein and in the claims, the following non-exclusive definitions are provided.

In one example, one or more instructions or steps discussed herein are automated. The terms automated or automatically (and like variations thereof) mean controlled operation of an apparatus, system, and/or process using computers and/or mechanical/electrical devices without the necessity of human intervention, observation, effort and/or decision.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub combination.

The applicant hereby gives notice that new claims may be formulated to such features and/or combinations of such features during the prosecution of the present application or of any further application derived therefrom.

For the sake of completeness it is also stated that the term "comprising" does not exclude other elements or steps, the term "a" or "an" does not exclude a plurality, a single processor or other unit may fulfil the functions of several means recited in the claims and reference signs in the claims shall not be construed as limiting the scope of the claims.

## Claims

1. A wearable leader sensor node for determining body posture, the wearable sensor leader node comprising:
a processor;
an orientation sensor coupled to the processor;
a memory coupled to the processor;
an ultra-wide-band, UWB, transceiver coupled to the processor;
at least two antennas coupled to the UWB transceiver;
wherein the processor is configured to:
transmit a first UWB signal via the ultra-wide band transceiver to a wearable follower sensor node;
receive a second UWB signal from the follower sensor node transmitted in response to receiving the first UWB signal;
determine a time-of-flight value of a signal transmitted between the wearable leader sensor node and the wearable follower sensor node from the first UWB signal and the second UWB signal;
determine an angle of arrival value of the second UWB signal; and
determine a value representing a body posture from the time-of-flight value and the angle-of-arrival value.

2. The wearable sensor leader node wherein the processor is configured to determine the body posture by comparison of the time-of-flight value and the angle of arrival value with a predetermined time-of-flight value and angle-of-arrival value stored in the memory.

3. The wearable sensor leader node of any preceding claim wherein the processor is configured for each follower node to:
transmit the first UWB signal via the ultra-wide band transceiver comprising a follower node ID;
receive a second UWB signal from the follower sensor node having the follower node ID;
determine a time-of-flight value of a signal transmitted between the wearable leader sensor node and the follower sensor node from the first UWB signal and the second UWB signal;
determine an angle of arrival of the second UWB signal from the wearable follower sensor node; and
determine a value representing a body posture from the time-of-flight values and the angle-of-arrival values determined between the sensor leader node and the follower nodes.

4. The wearable sensor node of any preceding claim wherein the angle of arrival is determined with respect to a reference axis determined from the orientation sensor.

5. The wearable sensor leader node of any preceding claim wherein the processor is further configured in a calibration step for each body posture to store the time-of-flight values and the angle-of arrival values in the memory.

6. The wearable sensor leader node of any preceding claim further configured to determine a user action from a comparison of the measured time-of-flight values and the angle-of arrival values with reference time-of-flight values and the angle-of arrival values.

7. The wearable sensor leader node of any preceding claim further comprising: a radio frequency, RF, transceiver coupled to the processor wherein the processor is further configured to transmit the determined body posture value via the RF transceiver to a further device configured to indicate to a user a required change in body posture.

8. The wearable sensor leader node of claim 7 wherein the further device is configured to indicate the required change in body posture from a difference between a measured angle-of-arrival value and a predetermined angle-of-arrival value.

9. A posture measurement apparatus comprising the wearable leader sensor node of any preceding claim wirelessly coupled to a plurality of wearable follower sensor nodes, each wearable follower sensor node comprising:
a processor;
a memory coupled to the processor;
an ultra-wide-band, UWB, transceiver coupled to the processor;
an antenna coupled to the UWB transceiver;
wherein the wearable follower sensor node processor is configured to:
receive a first UWB signal via the ultra-wide band transceiver from the wearable leader sensor node;
transmit a second UWB signal to the wearable leader sensor node in response to receiving the first UWB signal.

10. In a posture measurement system comprising a wearable sensor leader node, the wearable sensor leader node comprising a UWB transceiver coupled to at least two antennas and one or more wearable sensor follower nodes each comprising a UWB transceiver coupled to one antenna, a method of determining a body posture comprising:
transmitting a first UWB signal from the wearable sensor leader node to the one or more wearable follower sensor nodes;
receiving a second UWB signal by the wearable sensor leader node from each follower sensor node transmitted in response to receiving the first UWB signal;
determining a time-of-flight value of a signal transmitted between the wearable leader sensor node and the wearable follower sensor node from the first UWB signal and the second UWB signal;
determining an angle of arrival of the second UWB signal; and
determining a value representing a body posture from the time-of-flight value and the angle-of-arrival value.

11. The method of claim 10 further comprising determining the body posture value by comparison of the time-of-flight value and the angle of arrival value with a predetermined time-of-flight value and angle-of-arrival value.

12. The method of claim 11 further comprising:
transmitting the first UWB signal from the wearable leader sensor node to a plurality of wearable follower sensor nodes;
receiving by the wearable leader sensor node a plurality of second UWB signals from each of the follower sensor nodes transmitted in response to receiving the first UWB signal by each of the respective follower sensor nodes;
determining a time-of-flight value of a signal transmitted between the wearable leader sensor node and each wearable follower sensor node from the first UWB signal;
determining an angle of arrival of the second UWB signal from each of the wearable follower sensor nodes; and
determining a value representing a body posture from the time-of-flight values and the angle-of-arrival values.

13. The method of claim 11 or 12 wherein the leader sensor node comprises a orientation sensor and the method further comprises:
determining a reference axis form the orientation sensor;
and determining an angle of arrival with respect to the reference axis.

14. The method of any of claims 11 to 13 further comprising in a calibration step determining reference values of the time-of-flight and the angle-of arrival.

15. The method of claim 14 further comprising determining a user action from a comparison of the measured time-of-flight values and the angle-of arrival values with the reference time-of-flight values and the reference angle-of arrival values.
